# EUROPEAN PATENT APPLICATION

(11) **EP 4 483 921 A1**
(43) Date of publication of application: **01.01.2025**
(21) Application number: 22928996.2
(22) Date of filing: 23.02.2022
(51) Int. Cl.: A61M 5/145, A61M 5/155, A61M 5/14, A61J 1/06

(54) **METHOD FOR MANUFACTURING PRE-FILLED MEDICINAL LIQUID PUMPING ASSEMBLY, PRE-FILLED MEDICINAL LIQUID PUMPING ASSEMBLY, AND PRE-FILLED MEDICINAL LIQUID INJECTION SET**

(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2022/002641
(87) International publication number: WO 2023/163243

(57) **Abstract**

A method for manufacturing a pre-filled medicinal liquid pumping assembly according to a disclosed embodiment includes: a plunger disposing step in which a lubricant is applied to an inner surface of the chamber that defines an internal space connected to an injection port and the plunger is disposed inside the chamber such that the internal space has a predetermined volume; and a medicinal liquid pre-filling step in which the internal space of the chamber is filled with a medicinal liquid after the plunger disposing step.

## Description

### TECHNICAL FIELD

The present disclosure relates to a method for manufacturing a pre-filled medicinal liquid pumping assembly, a pre-filled medicinal liquid pumping assembly, and a pre-filled medicinal liquid injection set.

### BACKGROUND

In order to supply a medicinal liquid to a patient, a medicinal liquid injection apparatus configured to inject the medicinal liquid (e.g., an injection solution) in a liquid state into the patient is known. Using the medicinal liquid injection apparatus, the medicinal liquid in a predetermined storage space passes through a passage (e.g., the internal spaces of a tube and an injection needle) connected to a patient and is introduced into the body of the patient.

A conventional medicinal liquid injection apparatus injects a medicinal liquid through a process of expansion and contraction. Specifically, when the medicinal liquid is filled into the medicinal liquid injection apparatus, the space filled with the medicinal liquid expands and a pressure is generated to contract the space filled with the medicinal liquid. As a result, the medicinal liquid within the medicinal liquid injection apparatus can be injected into the patient by energy to return the space to its original volume. For example, there is known a medicinal liquid injection apparatus using compressive energy of an elastic body of a spring type, a balloon type, an air compression type or the like or air to generate a pressure for the above-mentioned contraction. In addition, a lubricant for sliding of the plunger may be applied to the inner surface of the chamber of the medicinal liquid injection apparatus.

In addition, in order to inject a medicinal liquid into a patient's body by using the medicinal liquid injection apparatus, a step of filling the medicinal liquid into the medicinal liquid injection apparatus is required. In a medical environment such as a hospital nowadays, since human and material movements frequently occur, the possibility of causing infection in the process of filling the medicinal liquid increases due to various unpredictable variables. In particular, since infections in the process of handling medicinal liquids may cause fatal results to patients, in order to prevent such infections, hospitals nowadays treat medicinal liquids according to specially managed procedures and regulations in sterile rooms. Due to the operation of sterile rooms and the safe treatment of medicinal liquids in hospitals, human and material costs are rapidly increasing.

### SUMMARY

E-WHA Biomedics Co., Ltd., a Korean company, has previously sold a product called "ANAPA," which is a medicinal liquid injection apparatus, and at least some embodiments of the present disclosure present more innovative apparatuses that are upgraded versions of the existing "ANAPA" apparatus.

In the conventional medicinal liquid injection apparatus, while a medicinal liquid is filled into the internal space of the chamber of the medicinal liquid injection apparatus, a plunger slides along the inner surface of the chamber, and the internal space is expanded at the same time. In this process, since the plunger slides along the inner surface of the chamber, the lubricant applied to the inner surface may be damaged or messily deformed. Thereafter, in the process in which the internal space is contracted for injection of the medicinal liquid, when the plunger slides once more along the same inner surface in a direction opposite to the moving direction in the expansion process, frictional resistance acting on the plunger is increased. In addition, it is very difficult to design the frictional resistance because the extent to which the lubricant is damaged or messily deformed cannot be controlled, and the frictional resistance varies depending on which portion of the inner surface the plunger is in contact with during the contraction process. Therefore, the deformation of the lubricant film makes it difficult to set a constant frictional force applied to the plunger during the contraction process, thereby causing a problem in that it is difficult to set a constant flow rate of the medicinal liquid. Embodiments of the manufacturing method of the present disclosure solve these problems of the prior art.

An embodiment of the present disclosure provides a medicinal liquid pumping assembly or a medicinal liquid injection set pre-filled with a medicinal liquid such that preparation work for medicinal liquid injection in a hospital, which is a special environment with a high risk of infection, can be reduced.

An embodiment of the present disclosure provides a safe and convenient pre-filled liquid pumping assembly or medicinal liquid injection set capable of minimizing human and material costs in a hospital.

In order to prevent the above-described deformation of a lubricant film, an aspect of the present disclosure provides embodiments of a method of manufacturing a pre-filled medicinal liquid pumping assembly. A method for manufacturing a pre-filled medicinal liquid pumping assembly according to a representative embodiment includes: a plunger disposing step in which a lubricant is applied to an inner surface of the chamber that defines an internal space connected to an injection port and the plunger is disposed inside the chamber such that the internal space has a predetermined volume; and a medicinal liquid pre-filling step in which the internal space of the chamber is filled with a medicinal liquid after the plunger disposing step.

Another aspect of the present disclosure provides embodiments of a pre-filled medicinal liquid pumping assembly. A pre-filled medicinal liquid pumping assembly according to a representative embodiment includes: a chamber having an internal space filled with a medicinal liquid and an injection port connected to the internal space; a plunger configured to be movable inside the chamber such that a volume of the internal space is reduced; and a leak-proof cap coupled to the chamber to block the injection port.

Another aspect of the present disclosure provides embodiments of a pre-filled medicinal liquid injection set. A pre-filled medicinal liquid injection set according to a representative embodiment includes: the pre-filled medicinal liquid pumping assembly; and a connection cap including a spike configured to be capable of penetrating the leak-proof cap to be inserted into the injection port and having a connection flow path which forms an upstream end at a protruding end of the spike, wherein the connection cap is configured to be capable of coupling to the medicinal liquid pumping assembly.

In an embodiment, the pre-filled medicinal liquid injection set may further include a hanger coupled to a predetermined position of the pre-filled medicinal liquid injection set such that the injection port is directed upward by gravity when the hanger is hung outside.

In an embodiment of the manufacturing method, since the medicinal liquid pre-filling step is performed after the plunger disposing step, the plunger is prevented from sliding on the inner surface of the chamber during the filling of the medicinal liquid in the manufacturing process such that damage to the lubricant film applied to the inner surface of the camber can be minimized. Accordingly, the frictional force applied to the plunger during the above-described contraction process for injecting the medicinal liquid into a patient can be set to be constant, and the medicinal liquid can be injected into the patient at a set constant inflow rate.

According to the embodiments of the pre-filled medicinal liquid pumping assembly or the pre-filled medicinal liquid injection apparatus, a medicinal liquid preparation operation or a medicinal liquid filling operation in a hospital can be minimized so that safety and convenience can be significantly improved and human and material costs in the hospital can be minimized.

With the leak-proof cap of the pre-filled medicinal liquid pumping assembly according to an embodiment, leakage of a medicinal liquid can be prevented during the distribution and use of the pre-filled medicinal liquid pumping assembly.

With the hanger of the pre-filled medicinal liquid injection set according to an embodiment, when the pre-filled medicinal liquid injection set is used, the injection port of the chamber is always directed upward so that the use environment by a user (patient) can be made uniform, and the movement convenience of the patient can be improved.

### BRIEF DESCRIPTION OF THE DRAWINGS

FIG. 1 is a conceptual view illustrating an entire system of a pre-filled medicinal liquid pumping assembly according to a first embodiment of the present disclosure and a pre-filled medicinal liquid injection set 1 according to an embodiment of the present disclosure.
FIG. 2 is a cross-sectional view illustrating a pre-filled medicinal liquid pumping assembly according to a second embodiment of FIG. 1.
FIG. 3 is a flowchart illustrating a method of manufacturing a pre-filled medicinal liquid pumping assembly according to embodiments of the present disclosure.
FIGS. 4A to 4C illustrate cross-sectional views sequentially showing states of manufacturing a pre-filled medicinal liquid pumping assembly according to a first embodiment of the manufacturing method of FIG. 3.
FIGS. 5A to 5C illustrate cross-sectional views sequentially showing states of manufacturing a pre-filled medicinal liquid pumping assembly according to a second embodiment of the manufacturing method of FIG. 3.
FIGS. 6A to 6C illustrate cross-sectional views sequentially showing states of manufacturing a pre-filled medicinal liquid pumping assembly according to a third embodiment of the manufacturing method of FIG. 3.
FIGS. 7A to 7C illustrate cross-sectional views sequentially showing states of manufacturing a pre-filled medicinal liquid pumping assembly according to a fourth embodiment of the manufacturing method of FIG. 3.
FIG. 8 is an exploded perspective view of the pre-filled medicinal liquid injection set of FIG. 1.
FIG. 9 is a cross-sectional view illustrating a medicinal liquid pumping assembly, a leak-proof cap, and a connection cap of the pre-filled medicinal liquid injection set of FIG. 1.
FIG. 10 is an enlarged partial cross-sectional view of a portion E of FIG. 9.
FIG. 11 is a partial cross-sectional view illustrating a state in which a spike 311 penetrates the leak-proof cap 200 in FIG. 10.
FIG. 12 is an enlarged perspective view of a portion of a chamber 110 of FIG. 8.
FIG. 13 is an elevational view of a portion of the chamber 110 of FIG. 12.
FIG. 14 is an elevational view illustrating a state in which the connection cap 310 is coupled to a portion of the chamber 110 of FIG. 12.

### DETAILED DESCRIPTION

Embodiments of the present disclosure are exemplified for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of rights according to the present disclosure.

As used in the present disclosure, expressions such as "comprising," "including," "having," and the like are to be understood as open-ended terms having the possibility of encompassing other embodiments, unless otherwise mentioned in the phrase or sentence containing such expressions.

The singular form used in the present disclosure may include a plural meaning unless otherwise mentioned. This equally applies to the singular form recited in the claims.

Terms such as "first" and "second" used in the present disclosure are used in order to distinguish a plurality of components from one another and do not limit the order or importance of the corresponding components.

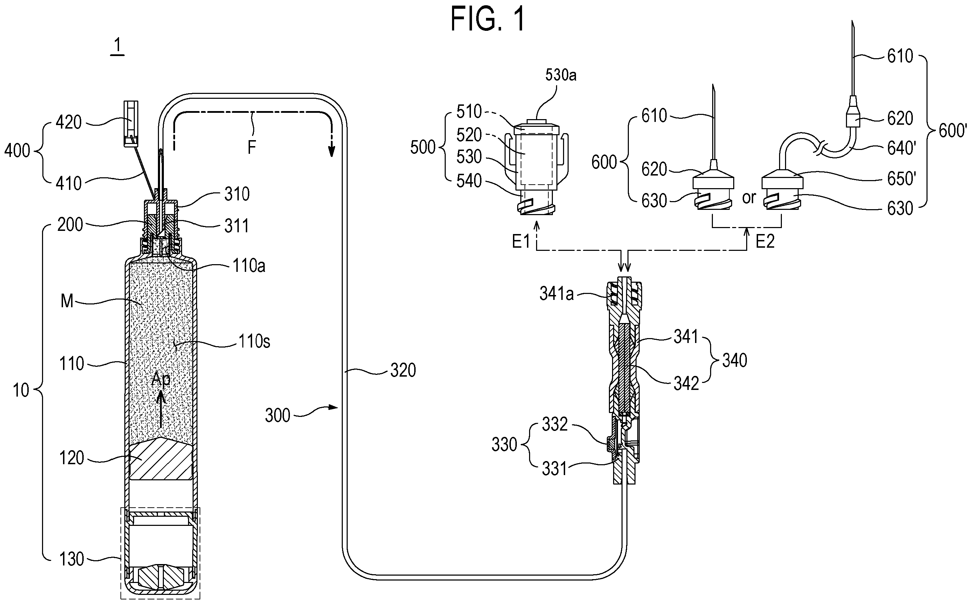

As used in the present disclosure, "upstream" and "downstream" are defined based on the direction in which a medicinal liquid flows when the plunger 120 presses the medicinal liquid. Specifically, the direction of arrow F in FIG. 1 is defined as the downstream direction, and the opposite direction to the downstream direction is defined as the upstream direction.

Hereinafter, embodiments of the present disclosure will be described with reference to the accompanying drawings. In the accompanying drawings, like or relevant components are indicated by like reference numerals. In the following description of embodiments, repeated descriptions of identical or related components may be omitted. However, even if a description of a component is omitted, such a component is not intended to be excluded in an embodiment.

FIG. 1 is a conceptual view illustrating an entire system of a pre-filled medicinal liquid pumping assembly according to a first embodiment of the present disclosure and a pre-filled medicinal liquid injection set 1 according to an embodiment of the present disclosure. The pre-filled medicinal liquid injection set 1 according to embodiments of the present disclosure refers to a medicinal liquid injection set 1 in a state of being pre-filled with a medicinal liquid M. In addition, the pre-filled medicinal liquid pumping assembly 10 according to embodiments of the present disclosure refers to a medicinal liquid pumping assembly 10 in a state of being pre-filled with a medicinal liquid M.

Referring to FIG. 1, the pre-filled medicinal liquid injection set 1 includes a pre-filled medicinal liquid pumping assembly 10. The pre-filled medicinal liquid pumping assembly 10 includes a medicinal liquid M filled inside the medicinal liquid pumping apparatus 100.

The medicinal liquid M is accommodated in an internal space 110s of the pre-filled medicinal liquid pumping assembly 10. The pre-filled medicinal liquid pumping assembly 10 includes a chamber 110 in which the internal space 110s filled with the medicinal liquid M is formed. An injection port 110a connected to the internal space 110s is formed in the chamber 110. The medicinal liquid M in the internal space 110s may be discharged through the injection port 110a.

The pre-filled medicinal liquid pumping assembly 10 includes a plunger 120 configured to be movable inside the chamber 110 such that the volume of the internal space 110s decreases. The plunger 120 may press the medicinal liquid M inside the chamber 110 by moving in a predetermined pressing direction Ap.

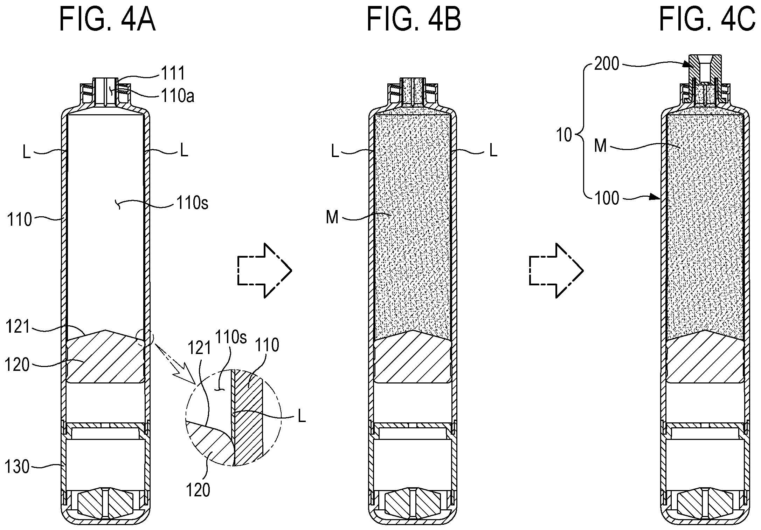

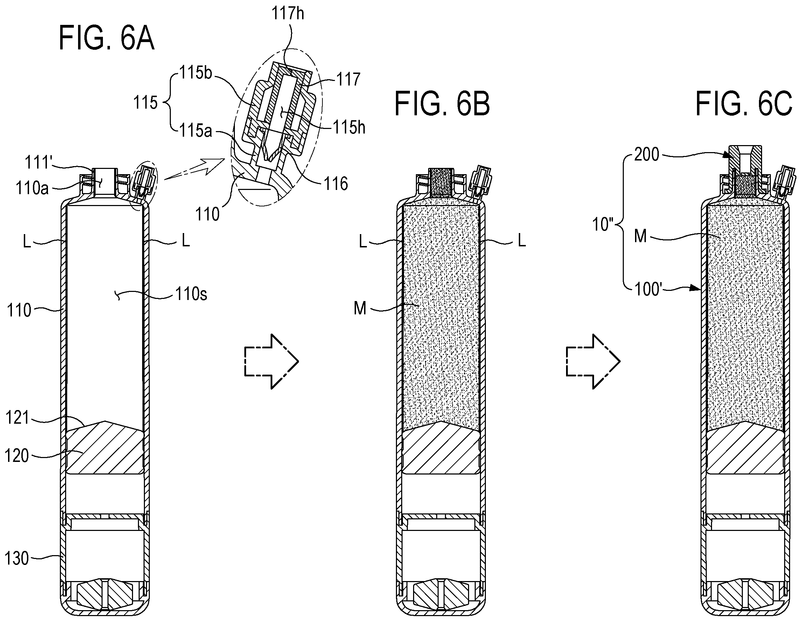

The pre-filled medicinal liquid pumping assembly 10 may further include a pressing actuator 130 configured to press the plunger 120 so as to move the plunger 120. For example, the pre-filled medicinal liquid pumping assembly 10 or 10" according to first and third embodiments of the present disclosure includes the pressing actuator 130 *(see* FIGS. 4A to 4C and 6A to 6C). The pressing actuator 130 may provide power so as to move the plunger 120 in the pressing direction Ap. As an example, the pressing actuator 130 may be configured to be capable of pressing the plunger 120 in the pressing direction Ap by a gas pressure generated in the pressing actuator 130 through gas activation, a balloon or the like. As another example, the pressing actuator 130 may move the plunger 120 in the pressing direction Ap with the force of an elastic body such as a spring. In the present embodiment, the pressing actuator 130 presses the plunger 120 by generating gas (e.g., carbon dioxide) in the pressing actuator 130 by gas activation.

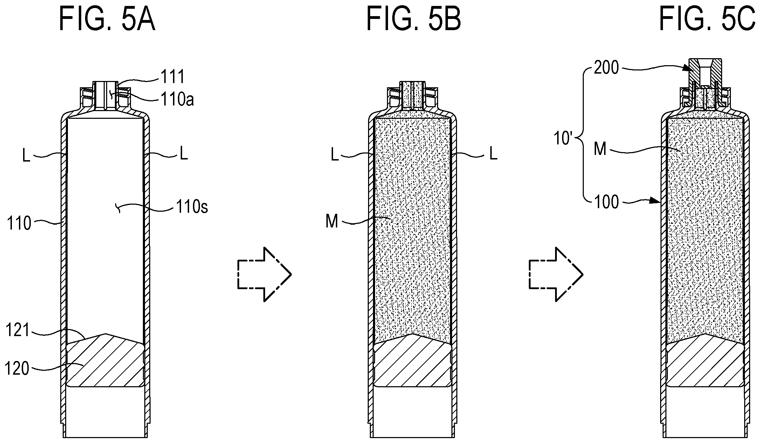

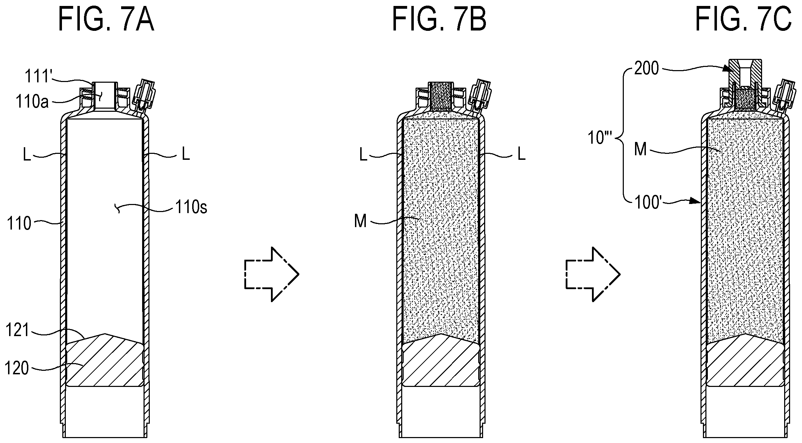

However, the pre-filled medicinal liquid pumping assembly may not include the pressing actuator 130. In this case, the pre-filled medicinal liquid pumping assembly which does not include the pressing actuator 130 may be manufactured, and users, such as pharmaceutical companies or a medical staff, may couple the pressing actuator 130 to the chamber 110 of the medicinal liquid pumping assembly by themselves. For example, the pre-filled medicinal liquid pumping assembly 10' or 10‴ according to second and fourth embodiments of the present disclosure does not include the pressing actuator 130 *(see* FIGS. 5A to 5C and 7A to 7C).

The pre-filled medicinal liquid pumping assembly 10 may include a leak-proof cap 200 coupled to the chamber 110. The leak-proof cap 200 is coupled to the chamber 110 to block the injection port 110a.

The pre-filled medicinal liquid injection set 1 may include a medicinal liquid flow section 300. The leak-proof cap 200 may be coupled to the medicinal liquid flow section 300. The medicinal liquid flow section 300 includes a medicinal liquid flow line 320. The medicinal liquid flow section 300 may include an air filter 330 and a medicinal liquid transfer tube device 340.

In order to inject the medicinal liquid into a patient by using the pre-filled medicinal liquid injection set 1, a priming process, which is a process for preparing medicinal liquid injection, and a medicinal liquid injection process may be performed. During the priming process, a priming liquid is allowed to flow along the medicinal liquid flow line 320. The priming process proceeds in a state in which an end cap 500 is coupled to the downstream end of the medicinal liquid flow section 300. The priming process is performed in a state in which the patient and the medicinal liquid injection set are separated. The priming liquid flowing along the medicinal liquid flow line 320 flows into the air filter 330 and a capillary device for the flow rate control 340 (e.g., a filter-integrated medicinal liquid transfer tube device). The pre-filled medicinal liquid injection set 1 may include an end cap 500 detachably connected to the downstream side of the medicinal liquid transfer tube device 340. The air inside the medicinal liquid flow line 320 may be discharged to the outside through the end cap 500. In the present embodiment, the medicinal liquid M inside the pre-filled medicinal liquid pumping assembly 10 may be used as the priming liquid.

The end cap 500 is configured such that the air passing through the medicinal liquid transfer tube device 340 and the priming liquid flow into the end cap 500. The end cap 500 may be configured to allow air to flow out to the outside but to prevent the priming liquid from flowing out to the outside.

The end cap 500 includes a vent filter 510 configured to block the passage of the priming liquid therethrough but to allow the passage of gas therethrough. The vent filter 510 includes a hydrophobic filter. The end cap 500 may include a sponge 520 disposed upstream of the vent filter 510. The end cap 500 includes an end cap casing 530 configured to accommodate the vent filter 510 in the end cap casing 530. The end cap casing 530 provides a vent hole 530a through which the gas passes. The end cap 500 includes an end cap coupler 540 configured to be capable of being coupled to a downstream connector 341a of the medicinal liquid transfer tube device 340. Arrow E1 in FIG. 1 illustrates the coupling and decoupling directions of the end cap coupler 540 with respect to the downstream connector 341a.

When the end cap 500 is filled with the priming liquid, the end cap 500 may be decoupled from the medicinal liquid transfer tube device 340, and the medicinal liquid transfer tube device 340 and a patient connection unit 600 or 600' may be connected to each other.

The patient connection unit 600 or 600' may include an injection needle 610, a catheter, or the like. The patient connection unit 600 or 600' includes a component which is introduced into a patient's body, such as an injection needle 610.

The patient connection unit 600 or 600' may include an "introduction component including a component to be introduced into the patient's body, such as the injection needle 610" and a "remaining component." The introduction component and the remaining component may be detachably coupled to each other. In this case, in the state in which the introduction component is connected to the patient but is decoupled from the remaining component, the user may couple the remaining component to the medicinal liquid transfer tube device 340 and may then couple the introduction component and the remaining component to each other. In this case, the liquid passing through the medicinal liquid transfer tube device 340 may flow into the patient's body after sequentially passing through the remaining component and the introduction component.

The patient connection unit 600 or 600' includes an injection support 620 configured to support the injection needle 610. The patient connection unit 600 or 600' includes a unit coupler 630 configured to be capable of coupling with a downstream connector 341a of the medicinal liquid transfer tube device 340. Arrow E2 in FIG. 1 illustrates the coupling and decoupling directions of the unit coupler 630 with respect to the downstream connector 341a.

As an example, the patient connection unit 600 may be configured by sequentially connecting the injection needle 610, the injection support 620, and the unit coupler 630.

As another example, the patient connection unit 600' further includes a patient connection tube anchor 650' connected to the downstream side of the unit coupler 630. The patient connection unit 600' further includes a patient connection tube 640' that interconnects the patient connection tube anchor 650' and the injection support 620. The patient connection tube 640' may be formed of a flexible material. The patient connection unit 600' may be configured by sequentially connecting the injection needle 610, the injection support 620, the patient connection tube 640', the patient connection tube anchor 650', and the unit coupler 630.

The medicinal liquid flow line 320 is configured to guide the flow of the priming liquid. The upstream end of the medicinal liquid flow line 320 is connected to the connection cap 310. For example, the medicinal liquid flow line 320 may be a flexible tube. In a medicinal liquid injection step according to an embodiment, with reference to arrow F, when the plunger 120 is moved in the pressing direction Ap, the medicinal liquid M is capable of passing through the connection cap 310, the medicinal liquid flow line 320, the air filter 330, and the medicinal liquid transfer tube device 340 in sequence.

The air filter 330 may include a filter casing 331 connected to the medicinal liquid flow line 320 and a filter 332 disposed within the filter casing 331. The filter 332 of the air filter 330 is able to filter out air bubbles.

The medicinal liquid transfer tube device 340 may include a transfer tube casing 341 connected to the air filter 330 and a medicinal liquid transfer tube 342 disposed within the transfer tube casing 341. The medicinal liquid transfer tube 342 may have a capillary flow path configured such that the medicinal liquid M flows therethrough. The medicinal liquid transfer tube 342 may have a function of maintaining constant the flow rate per hour of the medicinal liquid flowing along the medicinal liquid flow line 320.

In another embodiment (not illustrated), a T-Valve including a port for introducing a priming liquid from the outside may be provided on the medicinal liquid flow line 320. In this case, an opening/closing device (not illustrated) such as a clamp configured to open/close a portion of the medicinal liquid flow line may be provided. The T-valve forms a branch flow path branched from the medicinal liquid flow line 320 and connected to the port.

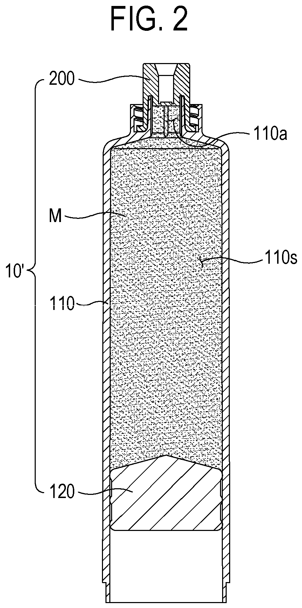

FIG. 2 is a cross-sectional view illustrating a pre-filled medicinal liquid pumping assembly according to a second embodiment of FIG. 1. Referring to FIG. 2, the pre-filled medicinal liquid pumping assembly 10' according to the second embodiment, unlike the pre-filled medicinal liquid pumping assembly 10 according to the first embodiment, does not include the pressing actuator. The pre-filled medicinal liquid pumping assembly 10' includes the chamber 110 in which the internal space 110s filled with the medicinal liquid M is formed, and the plunger 120.

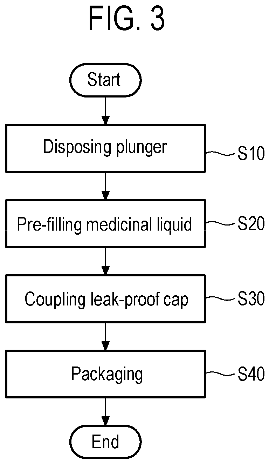

FIG. 3 is a flowchart illustrating a method of manufacturing a pre-filled medicinal liquid pumping assembly according to embodiments of the present disclosure. FIGS. 4A to 4C illustrate cross-sectional views sequentially showing states of manufacturing the pre-filled medicinal liquid pumping assembly 10 according to a first embodiment. FIGS. 5A to 5C illustrate cross-sectional views sequentially showing states of manufacturing a pre-filled medicinal liquid pumping assembly 10' according to a second embodiment of the manufacturing method. FIGS. 6A to 6C illustrate cross-sectional views sequentially showing states of manufacturing a pre-filled medicinal liquid pumping assembly 10" according to a second embodiment. FIGS. 7A to 7C illustrate cross-sectional views sequentially showing states of manufacturing a pre-filled medicinal liquid pumping assembly 10‴ according to a fourth embodiment of the manufacturing method.

As described above, the pre-filled medicinal liquid pumping assembly 10 or 10" which includes the pressing actuator 130 coupled to the chamber 110 may be manufactured (*see* FIGS. 4A to 4C and 6A to 6C), or the prefilled medicament pumping assembly 10' or 10‴ which does not includes the pressing actuator 130 may be manufactured (*see* FIGS. 5A to 5C and 7A to 7C).

Referring to FIGS. 6A to 7C, the pre-filled medicinal liquid pumping assembly 10" or 10‴ according to the third and fourth embodiments will be described focusing on differences from the pre-filled medicinal liquid pumping assembly 10 or 10' according to the first and second embodiments of FIGS. 4A to 5C.

Referring to FIGS. 6A to 7C, an injection port 110a and an inflow port 115h which are different from each other and are connected to the internal space 110s are formed in the chamber 110 of the pre-filled medicinal liquid pumping assembly 10" or 10‴. In the case of the pre-filled medicinal liquid pumping assembly 10" or 10'", since a separate inflow port 115h different from the injection port 110a can be used at the time of filling the internal space 110s with the medicinal liquid during the manufacturing process, an extension groove 111a in an injection port formation portion 111 of the chamber to be described later may not be formed in an injection port formation portion 111' of the chamber.

The chamber 110 of the pre-filled medicinal liquid pumping assembly 10" or 10‴ includes an inflow port formation portion 115 in which the inflow port 115h is formed. The inflow port forming portion 115 may include a first portion 115a fixed to one side of the chamber 110 and a second portion 115b fixed to the first portion 115a. The inflow port 115h may be formed through the first portion 115a and the second portion 115b.

The pre-filled medicinal liquid pumping assembly 10" or 10‴ may include a one-way valve 116 disposed in the inflow port 115h. The one-way valve 116 performs the function of a check valve. The one-way valve 116 prevents a reverse flow of the medicinal liquid M from the inflow port 115h to the outside of the internal space 110s. The one-way valve 116 allows the flow of the medicinal liquid moving from the outside to the internal space 110s (inflow) but blocks the flow of the medicinal liquid M moving from the internal space 110s to the outside (outflow). The one-way valve 116 may include a protrusion (not illustrated) protruding in the direction of the inflow, and a hole (not illustrated) may be formed at a protruding end of the protrusion. The hole of the one-way valve 116 is opened or closed depending on the flow direction of the medicinal liquid in the inflow port 115h. The hole of the one-way valve 116 is opened when the medicinal liquid M in the inflow port 115h flows in the inflow direction, and the hole of the one-way valve 116 is closed when there is no flow of the medicinal liquid M in the inflow port 115h or the medicinal liquid M tends to flow in the outflow direction. The one-way valve 116 may include a flange (not illustrated) seated by being sandwiched between the first portion 115a and the second portion 115b. The one-way valve 116 may be formed of a flexible material.

The pre-filled medicinal liquid pumping assembly 10" or 10‴ may include a pressing valve 117' configured to change whether the inflow port 115h is opened or closed. For example, the pressing valve 117' may be a swabable valve. The second portion 115b may support the pressing valve 117'. The pressing valve 117' may include a surface forming a hole 117h' which is opened when the pressing valve 117' is pushed from the outside. When a tip of a syringe is pressed from the outside to the surface forming the hole 117h' of the pressing valve 117', the surface may be bent to open the hole 117h'. The pressing valve 117' may be formed of a flexible material.

Referring to FIGS. 3 to 7C, the manufacturing method includes a plunger disposing step S10 of disposing the plunger 120 inside the chamber 110. A cross-sectional view of each of FIGS. 4A, 5A, 6A, and 7A illustrates a pre-filled medicinal liquid pumping assembly under manufacture in a state in which the plunger disposing step S10 is completed.

In the plunger disposing step S10, a lubricant L is applied to the inner surface of the chamber 110 defining the internal space 110s to which the injection port 110a is connected. In the plunger disposing step S10, the plunger 120 is disposed inside the chamber 110 such that the internal space 110s has a predetermined volume. In the plunger disposing step S10, the plunger 120 may be disposed inside the chamber 110 after applying the lubricant L to the inner surface of the chamber 110. Through this, the lubricant L applied to the inner surface of the chamber 110 may be maintained without being damaged or deformed.

The manufacturing method includes a medicinal liquid pre-filling step S20 in which the medicinal liquid is filled into the internal space 110s of the chamber 110. The medicinal liquid pre-filling step S20 proceeds after the plunger disposing step S10. A cross-sectional view of each of FIGS. 4B, 5B, 6B, and 7B illustrates a pre-filled medicinal liquid pumping assembly under manufacture in a state in which the medicinal liquid pre-filling step S20 is completed.

In the medicinal liquid pre-filling step S20, a medicinal liquid M may be filled into the internal space 110s in a state in which the plunger 120 is disposed at a position where the plunger 120 defines the internal space 110s of the predetermined volume such that the plunger 120 does not slide on the inner surface of the chamber 110.

In the medicinal liquid pre-filling step S20, the medicinal liquid M may be filled into the internal space 110s in a state in which the leak-proof cap 200 is removed from the chamber 110. Referring to FIGS. 4A to 7C, in an embodiment of manufacturing the pre-filled medicinal liquid pumping assembly 10, 10', 10", or 10‴, the medicinal liquid may be filled into the internal space 110s through the injection port 110a of the chamber 110.

Referring to FIGS. 6A to 7C, in an embodiment of manufacturing the pre-filled medicinal liquid pumping assembly 10" or 10'", the medicinal liquid may be filled into the internal space 110s through the inflow port 115h of the chamber 110. During the process of manufacturing the pre-filled medicinal liquid pumping assembly 10" or 10'", when the medicinal liquid is filled into the internal space 110s through the inflow port 115h, the air in the internal space 110s may be discharged through the injection port 110a.

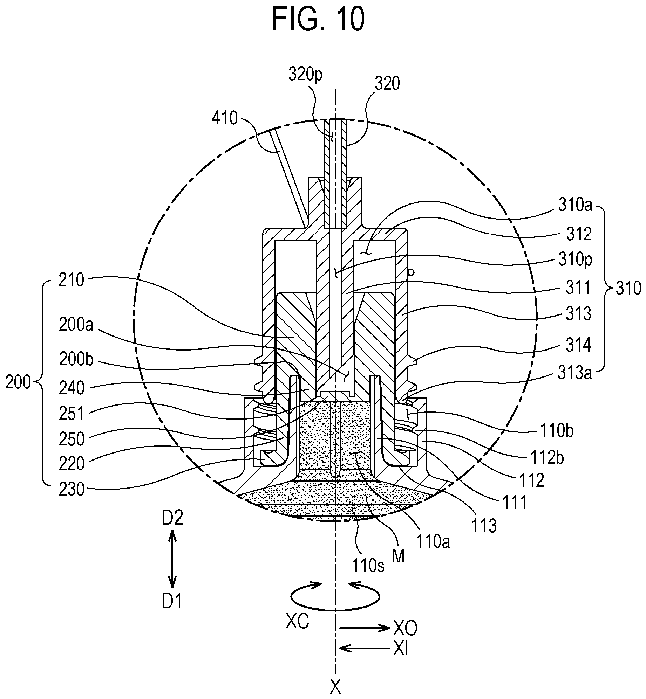

Referring to FIGS. 3 to 7C, the manufacturing method includes a leak-proof cap coupling step S30 of blocking the injection port 110a by coupling the leak-proof cap 200 to the chamber 110. The manufacturing method may include a packaging step S40 of packaging the pre-filled medicinal liquid pumping assembly 10, 10', 10", or 10'". In some embodiments, in the packaging step S40, only the pre-filled medicinal liquid pumping assembly 10, 10', 10", or 10‴ may be packaged, or the pre-filled medicinal liquid pumping assembly 10 or 10' and other components of the pre-filled medicinal liquid injection set 1 (e.g., the medicinal liquid flow section) may be packaged together. In addition, according to an embodiment, the pre-filled medicinal liquid pumping assembly 10, 10', 10", or 10‴ and the medicinal liquid flow section 300 may be packaged in the state of being separated from each other or may be packaged in the state of being temporarily coupled to each other (e.g., as illustrated in FIG. 10).

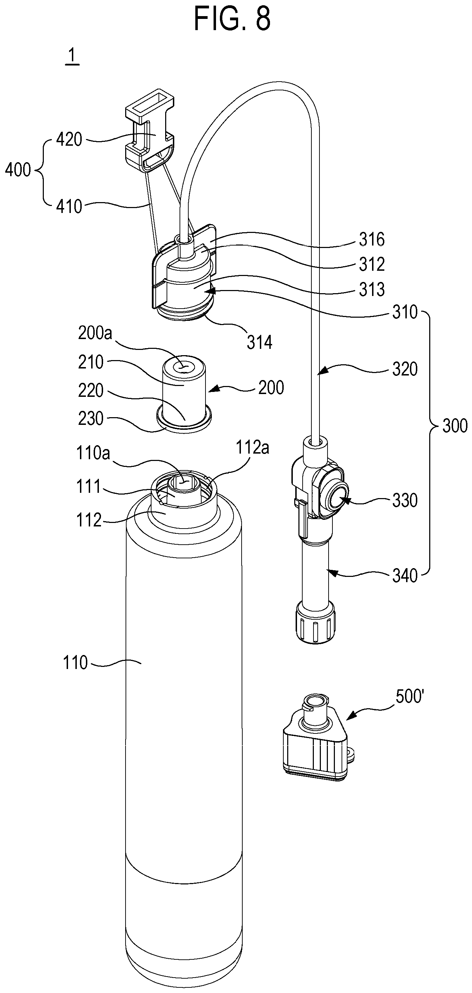

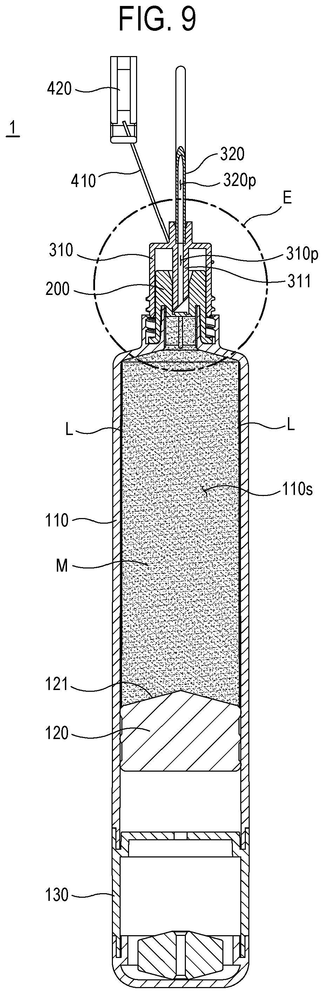

Hereinafter, the pre-filled medicinal liquid injection set 1 will be described in more detail with reference to FIGS. 8 to 14. FIG. 8 is an exploded perspective view of the pre-filled medicinal liquid injection set 1 of FIG. 1. FIG. 9 is a cross-sectional view illustrating the pre-filled medicinal liquid pumping assembly, the leak-proof cap 200, and the connection cap 310 of the pre-filled medicinal liquid injection set 1 of FIG. 1.

Referring to FIGS. 8 and 9, the plunger 120 includes a pressing surface 121 configured to pressure the medicinal liquid M in the internal space 110s. The pressing surface 121 defines one side surface of the internal space 110s. The leak-proof cap 200 may be disposed in the connection portion between the chamber 110 and the medicinal liquid flow section 300. The medicinal liquid flow section 300 may be configured to be capable of coupling to the chamber 110. The medicinal liquid flow section 300 may be coupled to both of the chamber 110 and the leak-proof cap 200.

The medicinal liquid flow section 300 may include a connection cap 310. The connection cap 310 may be configured to be capable of coupling to the medicinal liquid pumping assembly 10. The connection cap 310 may be configured to be capable of coupling to the chamber 110. The medicinal liquid flow line 320 is coupled to the connection cap 310. The medicinal liquid flow line 320 has a medicinal liquid flow path 320p connected to the downstream end of the connection flow path 310p of the connection cap 310.

The pre-filled medicinal liquid injection set 1 may include an end cap 500' detachably coupled to the downstream end of the medicinal liquid flow section 300. The end cap 500' may be detachably coupled to the downstream end of the medicinal liquid transfer tube device 340. Various types of end caps, such as the end cap 500 of FIG. 1 and the end cap 500' of FIG. 8, are known, and any one of them may be applied to the pre-filled medicinal liquid injection set 1.

The pre-filled medicinal liquid injection set 1 may include a hanger 400 configured to be hung outside. The hanger 400 may be coupled to a predetermined position of the pre-filled medicinal liquid injection set 1 such that the injection port 110a of the chamber 110 is directed upward by gravity when the hanger 400 is hung outside. In the present embodiment, the hanger 400 may be coupled to the connection cap 310 to support the pre-filled medicinal liquid injection set 1.

In an embodiment, the hanger 400 may include a ring 410 having one end fixed to the connection cap 310. The hanger 400 may include a ring connector 420 fixed to the other end of the ring 410. The hanger 400 may include an additional ring (not illustrated) configured to be detachably coupled to the ring connector 420. The additional ring may be hung on an external object such that the pre-filled medicinal liquid injection set 1 is hung.

Referring to FIG. 9, the pre-filled medicinal liquid injection set 1 may include a lubricant L applied to the inner surface of the chamber 110. The lubricant L may be applied to the inner surface of the chamber 110 in which the plunger 120 is slidable while moving. The lubricant L may be applied to the inner surface defining the internal space 110s of the chamber 110. For example, the lubricant L may be silicone oil or the like. The lubricant L is applied and cured on the inner surface of the chamber 110 to maintain a resistance value constant when the plunger 120 slides in the pressing direction *(see* arrow Ap in FIG. 1) along the inner surface of the chamber 110, thereby preventing or reducing the occurrence of factors that change the flow velocity of the medicinal liquid.

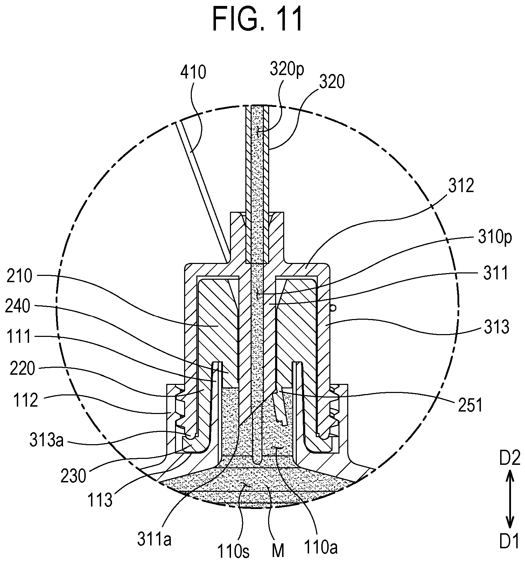

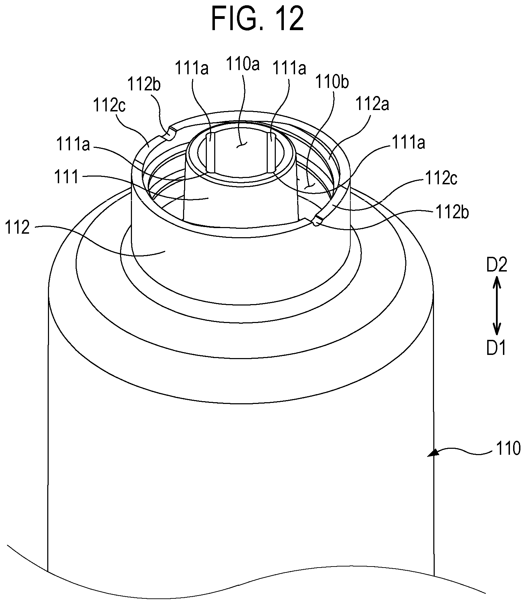

FIG. 10 is an enlarged partial cross-sectional view of a portion E of FIG. 9. FIG. 11 is a partial cross-sectional view illustrating a state in which a spike 311 penetrates the leak-proof cap 200 in FIG. 10. FIG. 12 is an enlarged perspective view of a portion of the chamber 110 of FIG. 8.

Referring to FIGS. 10 to 12, a "central axis" to be referred to below means an imaginary axis extending along the protruding direction of the injection port formation portion 111 of the chamber 110. Here, the central axis is an imaginary axis for explaining the present disclosure and does not refer to an actual component of the device. Hereinafter, the direction away from the central axis is defined as an "outer radial direction," the direction closer to the central axis is defined as an "inner radial direction," and the direction of rotation around the central axis is defined as a "circumferential direction." In addition, a direction opposite to the protruding direction of the injection port formation portion 111 is defined as a "first direction," and a direction opposite to the first direction is defined as a "second direction." This is merely for describing the present disclosure so that the present disclosure can be clearly understood, and each of the directions may be defined differently depending on where the reference is placed. FIG. 10 illustrates the central axis X, the outer radial direction XO, the inner radial direction XI, the circumferential direction XC, the first direction D1, and the second direction D2.

The connection cap 310 may be configured to have a temporarily coupled state in which the connection cap 310 is coupled to the leak-proof cap 200 and the spike 311 of the connection cap 310 does not penetrate the leak-proof cap 200 (*see* FIG. 10), and an injection-coupled state in which the connection cap 310 is located at a position shifted in the first direction D1 from the position of the connection cap 310 in the temporarily coupled state, such that the spike 311 penetrates the leak-proof cap 200 (*see* FIG. 11).

Referring to FIGS. 10 and 11, the connection cap 310 may include a spike 311 configured to be capable of penetrating the leak-proof cap 200 to be inserted into the injection port 110a. The connection cap 310 may be configured to be coupled to the chamber while covering the leak-proof cap 200. When using the pre-filled medicinal liquid injection set 1, the user may push the connection cap 310 in the first direction D1 so that the spike 311 can penetrate the leak-proof cap 200 *(see* FIG. 11). The user may rotate the connection cap 310 in the circumferential direction XC while pushing the connection cap 310 in the first direction D1 so that the second screw 112a of the chamber 110 and the first screw 314 of the connection cap 310 can be fastened to each other. The user may connect the chamber 110 and the medicinal liquid flow section 300 at once in this one-touch manner. The leak-proof cap 200 may serve as a medium for connecting the medicinal liquid flow section 300 and the chamber 110 to each other. In addition, the leak-proof cap 200 may implement a sealing function to prevent leakage of the medicinal liquid between the injection port 110a and the connection cap 310.

Referring to FIGS. 10 to 12, the chamber 110 may include an injection port formation portion 111 in which the injection port 110a is formed. The injection port formation portion 111 may protrude to the outside of the chamber 110. The injection port formation portion 111 may protrude in the second direction D2. The injection port formation portion 111 may surround the periphery of the injection port 110a.

The chamber 110 may include a circumferential extension 112 spaced apart from the injection port formation portion 111 in the outer radial direction XO to form a gap 110b with the injection port formation portion 111. The circumferential extension 112 may extend in the circumferential direction XC. The chamber 110 may be configured such that the second direction D2 of the gap 110b is opened. The gap 110b may extend in the circumferential direction XC.

The chamber 110 may include a second screw 112a configured to guide a first screw 314 of the connection cap 310 when the connection cap 310 is coupled to the chamber 110. The second screw 112a may be formed on the circumferential extension 112 of the chamber 110. The second screw 112a may be formed on the surface of the circumferential extension 112 in the inner radial direction XI.

The chamber 110 may include a gap base surface 113 defining the gap 110b in the first direction D1. The end of the gap base surface 113 in the inner radial direction XI may be connected to the injection port formation portion 111. The end of the gap base surface 113 in the outer radial direction XO may be connected to the circumferential extension 112.

Referring to FIGS. 10 and 11, the leak-proof cap 200 is coupled to the chamber 110 to block the injection port 110a. The injection port formation portion 111 of the chamber 110 may be fitted into the leak-proof cap 200. The leak-proof cap 200 may be fitted into the connection cap 310 to fix the connection cap 310. The leak-proof cap 200 may be formed of a flexible material such as rubber or silicon.

In the leak-proof cap 200, a spike insertion groove 200a recessed in a direction of insertion into the injection port 110a (*i.e.,* in the first direction D1) may be formed. The spike 311 of the connection cap 310 may be surrounded by the leak-proof cap 200 by being inserted into the spike insertion groove 200a. Through this, the leak-proof cap 200 prevents the spike 311 from being exposed to external air, thereby preventing a contamination source from flowing into the medicinal liquid.

The leak-proof cap 200 may include a cap body 210 formed around the spike insertion groove 200a. The spike insertion groove 200a may be formed in the center of the cap body 210. The cap body 210 may protrude in the second direction D2. The cap body 210 may be inserted into the cap insertion groove 310a of the connection cap 310.

The leak-proof cap 200 may include a partition 220 surrounding the injection port formation portion 111 of the chamber 110. The partition 220 may protrude from the cap body 210 in the first direction D1 and may extend in the circumferential direction XC. The partition 220 may be inserted into the gap 110b of the chamber 110. The partition 220 may extend in the first direction D1 to come into contact with the gap base surface 113 of the chamber 110.

The leak-proof cap 200 may include a flange 230 extending from the partition 220 in the outer radial direction XO. The flange 230 may extend along the gap base surface 113 of the chamber 110. The flange 230 may extend in the circumferential direction XC. The flange 230 may be pushed by a pushing end portion 313a of the connection cap 310 in the injection-coupled state. The sealing function of the leak-proof cap 200 can be further improved through the flange 230.

The leak-proof cap 200 may include an injection port insertion portion 240 inserted into the injection port 110a of the chamber 110. The injection port insertion portion 240 may protrude from the cap body 210 in the first direction D1. The injection port insertion portion 240 may be spaced apart from the partition 220 in the inner radial direction XI.

A protruding end insertion groove 200b into which the protruding end of the injection port formation portion 111 of the chamber 110 is inserted may be formed in the leak-proof cap 200. The protruding end insertion groove 200b may be formed along the periphery of the injection port insertion portion 240. The protruding end insertion groove 200b may be formed by being recessed in the second direction D2.

The leak-proof cap 200 may include a membrane 250 configured to allow the spike 311 to penetrate therethrough. The membrane 250 may be disposed in the center of the injection port insertion portion 240. The membrane 250 may be connected to the end portion of the injection port insertion portion 240 in the first direction D1. The membrane 250 may be located inside the injection port 110a.

The membrane 250 may include an incision guide portion 251 having a thinner thickness in the first direction D1 than other portions of the membrane 250. The incision guide portion 251 may extend along a rotational trajectory centered on the axis of screw rotation of the tip 311a of the spike 311 in the first direction D1. Through this, when the user switches the pre-filled medicinal liquid injection set 1 to the injection-coupled state, a preset portion of the leak-proof cap 200 can be conveniently penetrated by the spike 311.

The connection cap 310 includes the spike 311 configured to be capable of penetrating the leak-proof cap 200 to be inserted into the injection port 110a. The spike 311 may protrude in parallel to the extension direction of the injection port 110a of the chamber 110. The protruding end of the spike 311 may form an inclined surface inclined at an acute angle with respect to the first direction D1. The connection cap 310 has a connection flow path 310p forming an upstream end at the protruding end of the spike 311. The connection flow path 310p extends along the spike 311. The downstream end of the connection flow path 310p is connected to the medicinal liquid flow path 320p of the medicinal liquid flow line 320.

A cap insertion groove 310a recessed in the second direction D2 may be formed in the connection cap 310. The spike 311 may be disposed in the cap insertion groove 310a.

The connection cap 310 may include a circumferential cover portion 313 surrounding the periphery of the partition 220 of the leak-proof cap 200. The circumferential cover portion 313 may be inserted into the gap 110b of the chamber 110.

The flange 230 of the leak-proof cap 200 may extend along the surface 113 of the chamber 110 in the outer radial direction XO from the partition 220. The circumferential cover portion 313 of the connection cap 310 may be configured to push the flange 230 in a direction toward the surface 113 of the chamber 110 when the connection cap 310 is coupled to the chamber 110 (*i.e.,* in the first direction D1). Through this, it is possible to reinforce the sealing function of the leak-proof cap 200.

In the injection-coupled state in which the spike 311 penetrates the leak-proof cap 200, the end 313a of the circumferential cover portion 313 in the first direction D1 may push the flange 230 of the leak-proof cap 200.

The connection cap 310 may include a first screw 314. The first screw 314 may be formed on the surface of the circumferential cover portion 313 in the outer radial direction XO.

The connection cap 310 may include a top cover portion 312 covering the surface of the leak-proof cap 200 in the second direction. The top cover portion 312 may support the circumferential cover portion 313 and the spike 311. The spike 311 may protrude from the top cover portion 312 in the first direction D1. The circumferential cover portion 313 may protrude from the top cover portion 312 in the first direction D1.

The connection cap 310 may include a lever 316 configured to allow a user to conveniently perform a screw rotation step (*see* FIG. 8). The lever 316 may protrude farther from the axis of screw rotation than other portions of the lever 316. The lever 316 may protrude from the circumferential cover portion 313 in the outer radial direction XO.

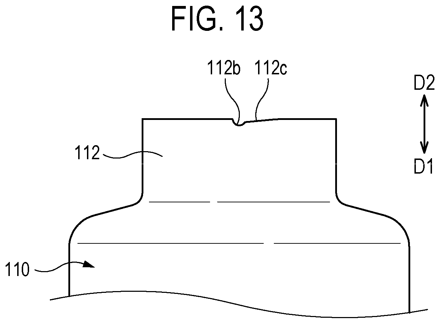

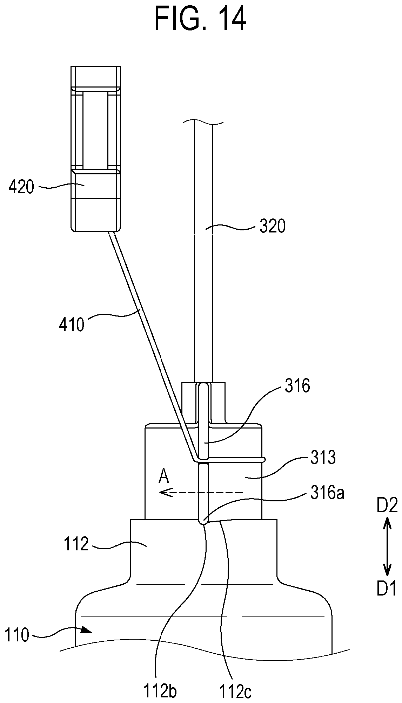

FIG. 13 is an elevational view of a portion of the chamber 110 of FIG. 12. FIG. 14 is an elevational view illustrating a state in which the connection cap 310 is coupled to a portion of the chamber 110 of FIG. 12. FIG. 14 illustrates the direction A of screw rotation by way of example.

Referring to FIGS. 12 to 14, an extension groove 111a extending along the protruding direction D2 of the injection port formation portion 111 may be formed on the surface of the injection port formation portion 111 in the inner radial direction XI. The extension groove 111a may extend to the protruding end of the injection port formation portion 111 in the second direction D2. Through this, even when a medicinal liquid injection nozzle is inserted into the injection port 110a to fill the internal space of the chamber 110 with a medicinal liquid when the pre-filled medicinal liquid injection set 1 is manufactured, the air in the internal space 110s can be smoothly discharged to the outside along the extension groove 111a.

One of the chamber 110 and the connection cap 310 may include an engagement groove recessed in a direction transverse to the direction A of rotation in which the first screw 314 is guided by the second screw 112a, and the other one of the chamber 110 and the connection cap 310 may include an engagement portion protruding to be engaged with the engagement groove in a state in which the spike 311 penetrates the leak-proof cap 200 (the injection-coupled state). Through this, the connection cap 310 may be designed to be difficult to separate from the chamber 110 after the chamber 110 and the connection cap 310 are locked. In the present embodiment, the engagement groove 112b is formed in the chamber 110, and the engagement portion 316a is formed in the connection cap 310.

The engagement groove 112b recessed in the first direction D1 may be formed on the surface of the circumferential extension 112 in the second direction D2. An engagement guide 112c recessed shorter in the first direction D1 than the engagement groove 112b may be formed on the surface of the circumferential extension 112 in the second direction D2. The engagement guide 112c may have an inclined surface in which the recessed depth in the first direction D1 increases along the direction A of the screw rotation. The engagement guide 112c may be connected to the engagement groove 112b in the direction A of the screw rotation. The engagement guide 112c may guide the sliding movement of the engagement portion 316a during the screw rotation of the connection cap 310 to guide the engagement portion 316a to be seated in the engagement groove 112b. The engagement portion 316a may be formed at the end of the lever 316 of the connection cap 310 in the first direction D 1.

The technical idea of the present disclosure has been described heretofore with reference to some embodiments and examples illustrated in the accompanying drawings. However, it is to be understood that various substitutions, modifications, and alterations may be made without departing from the technical idea and scope of the present disclosure that can be understood by those of ordinary skill in the technical field to which the present disclosure pertains. In addition, it is to be understood that such substitutions, modifications, and alterations fall within the scope of the appended claims.

## Claims

1. A method for manufacturing a pre-filled medicinal liquid pumping assembly comprising a chamber and a plunger, the method comprising:
a plunger disposing step in which a lubricant is applied to an inner surface of the chamber that defines an internal space connected to an injection port and the plunger is disposed inside the chamber such that the internal space has a predetermined volume; and
a medicinal liquid pre-filling step in which the internal space of the chamber is filled with a medicinal liquid after the plunger disposing step.

2. The method of Claim 1, wherein, in the medicinal liquid pre-filling step, the internal space is filled with the medicinal liquid in a state in which the plunger is disposed at a position defining the internal space by the predetermined volume such that the plunger does not slide on the inner surface of the chamber.

3. The method of Claim 1, wherein, in the plunger disposing step, the plunger is disposed inside the chamber after the lubricant is applied to the inner surface of the chamber.

4. The method of Claim 1, further comprising:
a leak-proof cap coupling step in which a leak-proof cap is coupled to the chamber to block the injection port.

5. A pre-filled medicinal liquid pumping assembly comprising:
a chamber having an internal space filled with a medicinal liquid and an injection port connected to the internal space;
a plunger configured to be movable inside the chamber such that a volume of the internal space is reduced; and
a leak-proof cap coupled to the chamber to block the injection port.

6. The pre-filled medicinal liquid pumping assembly of Claim 5, wherein the chamber comprises:
an injection port formation portion in which the injection port is formed and which protrudes outward;
a circumferential extension extending in a circumferential direction while being spaced apart from the injection port formation portion in an outer radial direction to form a gap with the injection port formation portion; and
a gap base surface defining the gap in a direction opposite to a protruding direction of the injection port formation portion,
wherein the leak-proof cap comprises a partition that surrounds a periphery of the injection port formation portion and is inserted into the gap.

7. The pre-filled medicinal liquid pumping assembly of Claim 6, wherein the leak-proof cap comprises a flange extending along the gap base surface in the outer radial direction from the partition.

8. The pre-filled medicinal liquid pumping assembly of Claim 5, wherein the leak-proof cap comprises:
an injection port insertion portion inserted into the injection port of the chamber; and
a membrane disposed in a central portion of the injection port insertion portion and configured to allow an external spike to penetrate therethrough.

9. The pre-filled medicinal liquid pumping assembly of Claim 8, wherein the chamber comprises an injection port formation portion in which the injection port is formed and which protrudes outward, and
wherein the leak-proof cap has a protruding end insertion groove which is formed along a periphery of the injection port insertion portion and into which a protruding end of the injection port formation portion is inserted.

10. The pre-filled medicinal liquid pumping assembly of Claim 5, wherein the leak-proof cap has a spike insertion groove recessed in a direction of insertion into the injection port.

11. A pre-filled medicinal liquid injection set comprising:
a pre-filled medicinal liquid pumping assembly according to Claim 5; and
a connection cap including a spike configured to be capable of penetrating the leak-proof cap to be inserted into the injection port and having a connection flow path which forms an upstream end at a protruding end of the spike, wherein the connection cap is configured to be capable of coupling to the medicinal liquid pumping assembly.

12. The pre-filled medicinal liquid injection set of Claim 11, further comprising:
a hanger coupled to a predetermined position of the pre-filled medicinal liquid injection set such that the injection port is directed upward by gravity when the hanger is hung outside.

13. The pre-filled medicinal liquid injection set of Claim 11, wherein the connection cap is configured to be capable of coupling to the chamber while covering the leak-proof cap.

14. The pre-filled medicinal liquid injection set of Claim 13, wherein the connection cap comprises a first screw,
wherein the chamber comprises a second screw configured to guide the first screw when the connection cap is coupled to the chamber,
wherein one of the chamber and the connection cap has an engagement groove recessed in a direction transverse to a direction of rotation in which the first screw is guided by the second screw, and
wherein the other one of the chamber and the connection cap includes an engagement portion protruding to be engaged with the engagement groove in a state in which the spike penetrates the leak-proof cap.

15. The pre-filled medicinal liquid injection set of Claim 11, wherein the chamber comprises an injection port formation portion in which the injection port is formed and which protrudes outward,
wherein the leak-proof cap comprises:
a partition surrounding a periphery of the injection port formation portion; and
a flange extending from the partition in an outer radial direction along a surface of the chamber, and
wherein the connection cap comprises a circumferential cover portion surrounding a periphery of the partition and configured to press the flange in a direction toward the surface of the chamber when the connection cap is coupled to the chamber.
